# EUROPEAN PATENT APPLICATION

(11) **EP 4 822 349 A1**
(43) Date of publication of application: **14.10.2026**
(21) Application number: 25382378.5
(22) Date of filing: 11.04.2025
(51) Int. Cl.: G01N 33/00, G01W 1/02, G01N 15/00

(54) **DEVICE FOR MONITORING AIR QUALITY**

(71) Applicant: Bettair Cities SL, 08907 L'Hospitalet de Llobregat (ES)
(72) Inventor: Perelló Vallès, Josep, 08849 Sant Climent de Llobregat (ES); Ramirez Javega, Francisco, 08028 Barcelona (ES); Santiago Montilla, Leonardo Alberto, 08812 Sant Pere de Ribes (ES); Udina Oliva, Sergi, 08901 L'Hospitalet de Llobregat (ES)
(74) Representative: Curell Suñol S.L.P.

(57) **Abstract**

Device (1) for monitoring air quality, comprising a housing (2) containing: at least one sensor, configured to obtain measurements of at least one component of the atmospheric composition related to air quality; a control means, configured to receive said measurements of said at least one sensor, and to obtain air monitoring data from said measurements; and a communication means, configured to send said air monitoring data to an external device,
the device (1) further comprising an external shelter (3) comprising: a roof (4); an outer wall (6), comprising four rectangular walls defining a cube or cuboid, said outer wall (6) enclosing an internal space (7) covered by said roof (4); and an internal support, provided inside said internal space (7), wherein each of said four rectangular walls comprises an external louver (8), and wherein said housing (2) is attached to said internal support.

## Description

### Field of the invention

The invention lies in the field of outdoor air quality monitoring technologies, and more particularly to devices designed for the measurement and analysis of air pollutants, particulate matter, aerosols, and/or greenhouse gases, or other components that are related to the general concept of air quality in a wide scope of definition.

In particular, the invention relates to a device for monitoring outdoor air quality, comprising a housing containing:
- at least one sensor, configured to obtain measurements of at least one component of the atmospheric composition related to air quality;
- a control means, configured to receive said measurements of said at least one sensor, and to obtain air monitoring data from said measurements; and
- a communication means, configured to send said air monitoring data to an external device.

### Prior art

This application addresses the problem of accurately monitoring air quality, for example, in cities, rural areas, industrial facilities or other environments. Examples of air quality monitoring include measuring gas pollutants or fine particles in the air generally referred as particulate matter, PM. However, in the context of this document, the concept of air quality has to be understood in a generic way, further including (but not limited to) possible measurements of greenhouse gases, aerosols, etc.

Devices for monitoring air quality are known in the art. This type of devices usually comprise one or more sensors encapsulated in a housing that further contains electronics for receiving the measurements of the sensors and send them to an external device, for example, a server. The aim of the housing is to protect the internal components from the weather conditions, in particular, from the rain. However, the protection is limited, and a trade-off between protection and accuracy has to be determined because the basic necessity of the device is that the air of the surrounding environment reaches the sensors with minimal alterations, turbulences or unwanted filtering. Otherwise, the readings of the sensors may not be accurate.

### Summary of the invention

The invention is aimed to provide a device for monitoring air quality of the type stated at the beginning, being able to avoid the problems that have been identified above.

This purpose is achieved by a device for monitoring air quality of the type indicated at the beginning, characterized in that the device further comprises an external shelter comprising:
- a roof;
- an outer wall, comprising four rectangular walls defining a cube or cuboid (sometimes named as *rectangular cuboid*), said outer wall enclosing an internal space covered by said roof; and
- an internal support, provided inside said internal space;
wherein each of said four rectangular walls comprises an external louver; and wherein said housing is attached to said internal support.

As it has been described above, in the context of this document, the term "air quality" may refer to different types of measures. In particular, gaseous air pollutants, particulate matter (including aerosols and ultra-fine particles), and greenhouse gas (GHG) emissions. Air pollutants include primary pollutants and secondary pollutants. A primary pollutant is an air pollutant emitted directly from a source such as: nitrogen dioxide (NO₂), nitrogen monoxide (NO), carbon monoxide (CO), sulphur dioxide (SO₂), hydrogen sulphide (H₂S), ammonia (NH₃), Volatile Organic Compounds (VOCs), particulate matter (PM), ultra fine particles and aerosols, etc. Fine particulate matter known as PM_{2.5} are particles with aerodynamic diameter lower than 2.5 µm, PM₁ measures particulate pollutants smaller than 1 µm while PM₁₀ are particles with a diameter lower than 10 µm. Airborne particles between 2.5 µm and 10 µm (PM₁₀ - PM_{2.5}) are also known as coarse particles. Examples of particulate matter include rubber and metal splinters from the wear and tear from tyres and brakes, dust, pollen, spores, fly ash, plant and insect parts. Ultra fine particles (UFPs), also known as PM_{0.1}, are airborne particles with and aerodynamic diameter of 0.1 µm or less. An aerosol is a collection of suspended particles and the surrounding gases. A secondary pollutant is not directly emitted as such, but when other pollutants (primary pollutants) react in the atmosphere, for example, ground-level ozone (O₃), sulphur trioxide (SO₃), ammonium (NH₄), sulfuric acid (H₂SO₄), hydrogen peroxide (H₂O₂), nitric acid (HNO₃), PMs composed of primary and secondary pollutants, etc. Regarding GHG emissions, the concept mainly includes carbon dioxide (CO₂), methane (CH₄), nitrous oxide (N₂O), hydrofluorocarbons (HFCs), perfluorocarbons (PFCs), sulphur hexafluoride (SF₆) and nitrogen trifluoride (NF₃).

The term "rectangular" used above has to be considered as also including the particular case of a square. The term does not exclude a rectangular outer wall having rounded corners.

The term "louver" refers to a structure having an external side and an internal side, formed by a horizontal pattern of blocking elements and openings. Said openings are arranged to allow the passage of air between the two sides of the louver. Said blocking elements are arranged for blocking the passage of sunlight, in particular, for blocking direct light beams coming from a range of directions. Therefore, direct light beams are blocked and/or scattered by means of the louver and only dim scattered light, or light beams coming from a small range of upwards direction angles (if any) can pass thought the louver, and even in this case, the eventual beams do not penetrate far inside the shelter so that they cannot impinge on the sensors. Since the sun is not located in a direction able to send upward light beams, the louver structure of each rectangular wall effectively avoids direct sunlight inside the shelter, as well as a range of sunlight reflected in nearby objects. A further effect of the blocking elements of the louver is deflecting raindrops. The roof acts as a further blocking element for the strict vertical direction, that is, blocking the sun when it is high in the sky and also blocking most part of the rain.

Even if the customary practice in the art is avoiding covering and/or encapsulating the sensors as much as possible, it has been surprisingly found that providing an additional shelter having the technical features described above actually may even improve the accuracy of the measurements. In particular, contrary to what may be expected, it has been found that the type of openings of a louver of the outer wall described above do not interfere with the components of the air to a degree that really affects the measurements of the sensors, usually in the range of parts-per-billion (ppb). A small impact may be present, but it is generally negligible. However, it has been found that avoiding the sunlight in the sensors substantially improves the accuracy and consistency of the measures, for example, by smoothing temperature changes due to temporal variations of the incident sunlight that could lead to imprecise temporal measurements, introducing non-linearities and also eventual thermal noise, that can change rapidly with time. This effect is particularly relevant depending on the weather conditions, for example, when there are rapid variations of the sunlight due to presence/absence of clouds. Moreover, changes in temperature may also affect the behaviour of the batteries (variations in their output and/or available charge) in those devices that are battery-powered. As has been discussed before, the combination of a low affectation in the components of the air by the louvers with the improvement achieved by blocking the sunlight have a synergistic effect that improves the overall accuracy of the measurements of the sensors and, as a consequence a more accurate and consistent air quality monitoring over time is achieved.

Preferably, each of said rectangular walls further comprises an internal louver, in particular, arranged in parallel with said external louver. The double-louver configuration improves blocking the light beams, in particular, if the inclination of the blocking elements of the internal louver is contrary to the inclination of the blocking elements of the external louver. This advantageous configuration allows an improved blockage of sunlight. Due to the double-louver configuration the device may be bulkier, and the air composition may be slightly more altered than in the case with a single louver, however, it has been found that the benefit obtained by the improved blockage of light may be more relevant, especially in environments with strong sunlight and/or wherein there could be hard reflections from surrounding objects.

Preferably, said measurements of at least one component of the atmospheric composition related to air quality by said at least one sensor comprise measurements of air pollutants, particulate matter, aerosols, and/or greenhouse gases. The capability to measure various components of atmospheric composition related to air quality allows for comprehensive monitoring and analysis of environmental conditions, which is critical for public health and regulatory compliance. By detecting air pollutants, particulate matter, aerosols and greenhouse gases, the device can contribute to the development of targeted strategies for pollution control and climate change mitigation, supporting efforts to improve air quality and reduce environmental impact.

Preferably, said at least one sensor comprises at least one of:
- an electrochemical sensor, configured to obtain measurements of at least one chemical species in the air, preferably at least one of NO₂, NO, CO, O₃, H₂S, SO₂, NH₃, and volatile organic compounds, VOCs;
- an optical particle counter or nephelometer, configured to obtain measurements of particulate matter, PM, in the air;
- a non-dispersive infrared sensor (NDIR), configured to obtain measurements at least one greenhouse gas in the air, preferably at least one of CO₂, CH₄ and N₂O;
- a photo ionization detector, PID, configured to obtain measurements of at least one volatile organic compound, VOC;
- a solid-state gas sensor;
- a MEMS gas sensor; and
- a photoacoustic gas sensor.

Preferably, said external device is one of:
- a server;
- a datalogger;
- a displaying device, configured for displaying said air monitoring data; and
- a retransmission device.

The aforementioned options provide flexibility in how the air monitoring data is stored, processed, and analysed, catering to different user needs and applications. The particular case of a server must be interpreted in a generic manner, including not only a physical server but any equivalent server system known in the art, as non-limiting examples, a cluster of servers, virtualized servers, etc. Connecting to a displaying device enables immediate visualization of air quality data, which may be necessary depending on the application of the device. Public displays may also be used for raising public awareness and for the implementation of timely protective measures in response to poor air quality conditions. The compatibility with retransmission devices extends the reach of the air quality monitoring system, allowing data to be shared across different platforms and users, which can be particularly beneficial for large-scale environmental monitoring networks.

Preferably, said external shelter comprises a further roof, provided over said roof. The external shelter with an additional roof provides enhanced protection against environmental elements such as rain, dust, snow, and direct sunlight, thereby increasing the durability and lifespan of the underlying structure. The further roof creates an air gap between the primary roof and the shelter, which can serve as an insulation layer to regulate the temperature within the internal space where the device is located, improving accuracy, durability and lifetime.

Preferably, said further roof is flat and arranged at a slant, has a pyramidal shape or a has conical shape. A flat roof arranged at a slant simplifies the manufacture and facilitates water runoff, reducing the likelihood of water accumulation and potential damage from standing water or heavy rainfall. The pyramidal or conical shape of the further roof offers structural stability and can distribute weight evenly, which is particularly beneficial in regions with heavy snowfall or high winds. In embodiments wherein the further roof is not present, the roof may have the features described here in other to obtain equivalent technical effects, for example, the roof may be flat and arranged at a slant, having a pyramidal shape or having a conical shape.

Preferably, the device further comprises a support for said external shelter, said support preferably comprising at least one leg and more preferably four legs, preferably said at least one leg having a length of 100 to 160 cm, in particular 120 cm. The inclusion of at least one leg, and preferably four, provides a stable foundation for the device, ensuring it remains upright and secure even on uneven terrain or in adverse weather conditions. The resulting device is separated from the ground, thereby avoiding the heat that may be radiating from said ground and affecting the accuracy of the measures of the sensors. Besides, the separation also protects the device from ground moisture, pests, and facilitate easier access for maintenance or usage.

Preferably, at least one of said four walls is hinged such that it can act as a door that is swingable between an opened position and a closed position; and locking means, as a non-limiting example one or more latches, said locking means configured for locking said wall when in said closed position.

The hinged wall that functions as a door enables convenient access to the interior of the device, allowing for easy loading, unloading, or entry and exit without the need for disassembly. The locking means may also be used for securing the wall in the closed position and enhance the security of the device, protecting contents from theft or tampering and ensuring that the door remains closed during transport or in windy conditions.

Preferably, said internal space has a height of 350 - 730 mm, a width of 230 - 700 mm, and a depth of 200 - 630 mm.

Preferably, said housing has a housing height, a housing width and a housing depth, and said internal space has a space height, a space width and a space depth, wherein said space height is two times said housing height, said space width is two times said housing width, and said space depth is two times said housing depth. Therefore, the internal space is two times the size of the housing in every dimension X, Y and Z. This embodiment allows for improved airflow and separation from the light that may pass through the outer wall, and the temperature of the outer wall, while keeping the overall size of the device controlled and not unnecessarily bulky

Preferably, said external shelter is made of at least one of wood, plywood, oriented strand board, particle board, fiberglass, and plastic. The use of materials such as wood, plywood, oriented strand board, particle board, fiberglass, and plastic for the external shelter provides versatility in construction, allowing for a balance between durability, cost-effectiveness, and ease of manufacturing. These materials can offer various degrees of resistance to environmental conditions, such as corrosion, UV radiation, and physical impacts, thereby enhancing the device's overall resilience and service life.

Preferably, said external shelter has an internal surface facing said internal space, and an external surface; and wherein said external surface is white or of a clear colour. The white or clear colour of the external surface can reflect sunlight and reduce heat absorption, contributing to thermal management and energy efficiency. Depending on where the device is installed, a white or clear external surface can also provide aesthetic benefits, allowing the device to blend into different environments or maintain a neutral appearance that is less visually intrusive.

Preferably, said external shelter has an internal surface facing said internal space; and wherein said internal surface is black or of a dark colour. The use of a black or dark-coloured internal surface within the external shelter can enhance the absorption of solar radiation, avoiding external reflections of the residual light that may have passed through the outer wall. The dark internal surface can reduce glare and light reflection within the internal space, thereby minimizing the amount of light that may reach the sensors.

Preferably, said external shelter further comprises a base floor. The base floor of the shelter can serve as an additional insulating barrier, contributing to the thermal regulation of the internal space by further isolating the sensors from the temperature of the ground and blocking the light that may be reflected on the ground.

Preferably, said external louver and, where appropriate, also said internal louver, are each formed by a plurality of strips arranged horizontally and having a roll inclination, with a vertical separation between strips. Preferably, in the case of a double-louver structure, the roll inclination of the internal louver strips is contrary to the roll inclination of the external louver strips. The strips function as blocking elements and the vertical separation between strips as the openings described above. This way, the louvers with horizontally arranged strips and roll inclination allow for customizable control of sunlight and airflow into the internal space with the aforementioned advantages. As a non-limiting example, the said external shelter is a weather instrument shelter of the type having louvered walls. Up to now, this type of shelters has only been used as a housing for meteorological instruments, for example, thermometers, hygrometers or barometers.

Preferably, said external shelter comprises an external louver and an internal louver, and wherein said horizontal strips of said internal louver have a roll inclination which is contrary to the roll inclination of said horizontal strips of said external louver. As it has been explained above, the opposing roll inclinations of the horizontal strips in the internal and external louvers can create a shading system that allows to block a wider range of angles of the incoming light beams, either direct light from the sun or light that has been reflected in nearby objects.

Preferably, said external shelter is provided with forced ventilation means, configured for forcing the circulation of air between the outside of said external shelter and said internal space. In particular environmental and weather conditions, for example, in situations wherein the external wind is very light or even not present, the inclusion of forced ventilation means in the external shelter may enhance the thermal management of the internal space, ensuring that electronic or mechanical components housed within maintain optimal operating temperatures, thereby improving reliability and longevity, independently of the outside wind conditions. The active ventilation system allows for the external shelter to be used in a wider range of environmental conditions, including areas with high ambient temperatures or stagnant air, thus increasing the versatility and deployment possibilities of the device.

The invention also includes other detail features illustrated in the detailed description of some embodiments of the invention and in the accompanying figures.

### Brief description of the figures

Further advantages and features of the invention will become apparent from the following description, in which, without any limiting character, preferred embodiments of the invention are disclosed, in reference to the accompanying figures:
Fig. 1 is a frontal view of a housing containing sensors, control means and communication means.
Fig. 2 is a lateral view of the housing of Fig. 1.
Fig. 3 is schematic representation in perspective of a device for monitoring air quality, comprising the housing of Fig. 1 and Fig. 2 inside an external shelter. For the sake of clarity in showing the other elements, the internal support is not shown.
Fig. 4 is a lateral view of the device of Fig. 3.
Fig. 5 is schematic representation in perspective of another exemplary embodiment of a device for monitoring air quality, wherein the external shelters comprises a further roof and a base. The internal support is not shown.
Fig. 6 is a detail view of a section of a louver of the outer wall of the external shelter.
Fig. 7 is a detail view of a section of a double louver of the outer wall.
Fig. 8 is a schematic representation in perspective of a device for monitoring air quality, comprising legs.

### Detailed description of embodiments of the invention

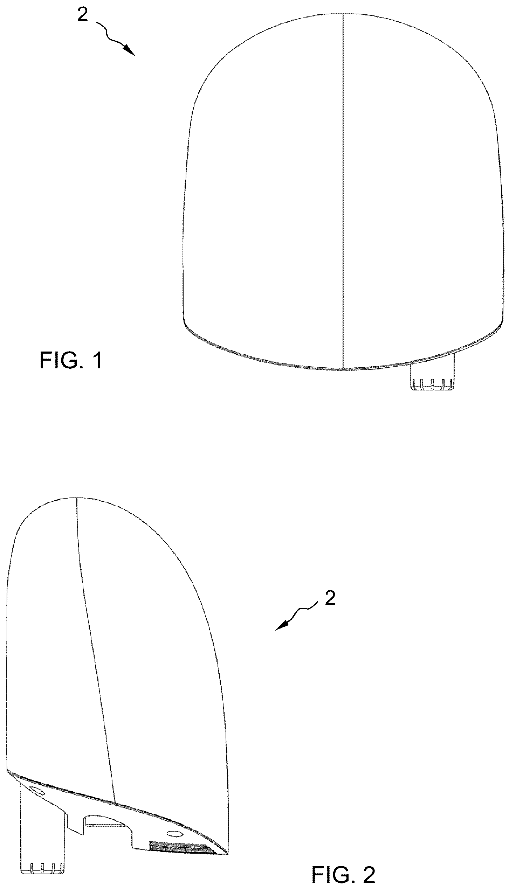

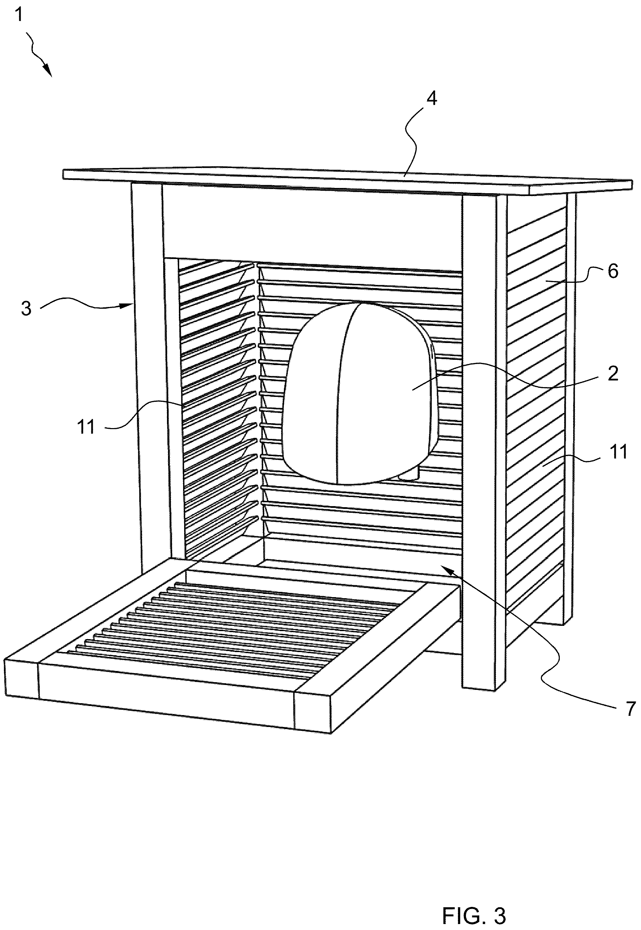

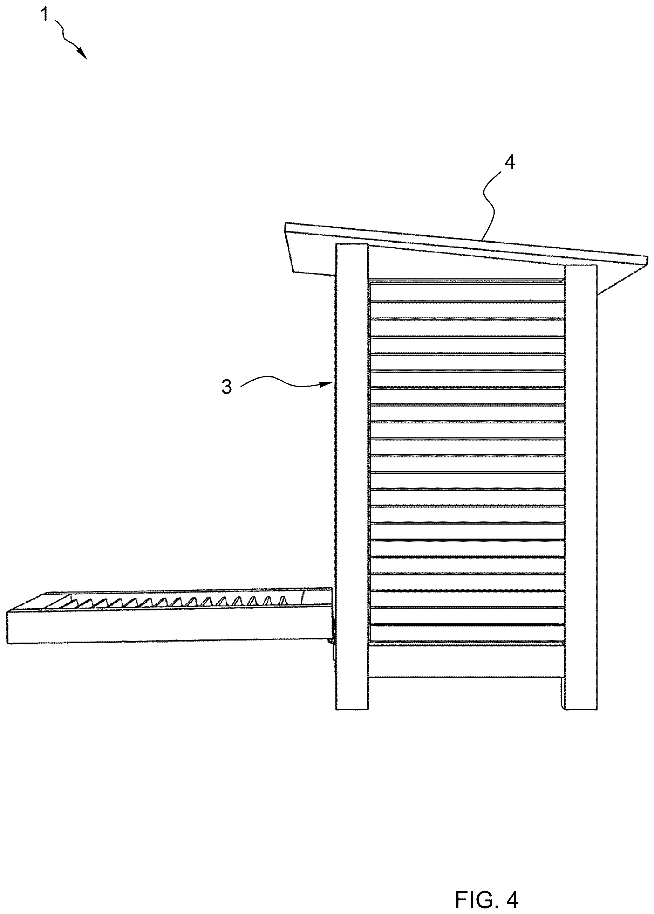

Fig. 3 and Fig. 4 show schematic simplified representations of a first embodiment of a device 1 for monitoring air quality according to the invention. The device 1 has an external shelter 3 and a housing as the one shown in Fig. 1 and Fig. 2. Said housing contains a plurality of sensors that are configured to obtain measurements several components of the atmospheric composition that are related to air quality. In the first exemplary embodiment, said measurements are measurements of air pollutants, particulate matter, and CO₂ which is a greenhouse gas. Other air quality related components may also be envisaged, for example, the presence of aerosols or other greenhouse gases like CH₄ or N₂O. In order to obtain said measurements, the housing 2 comprises a plurality of electrochemical sensors for measuring NO₂, NO, CO, O₃, H₂S, SO₂, NH₃, and volatile organic compounds, VOCs, an optical particle counter, to obtain measurements of particulate matter, PM₁, PM_{2.5} and PM₁₀, and an infrared sensor to obtain measurements of CO₂.

A communication means are also provided and configured to send said air monitoring data to a server. In the first exemplary embodiment, the communication means are 4G communication, but other wireless or even wired communication means known in the art may be envisaged. In other embodiments, the monitoring data is sent to other type of external device, for example, a datalogger, a retransmission device or a displaying device that allows displaying the air monitoring data that is received.

As shown schematically in Fig. 3, the housing 2 comprising all the internal elements is arranged inside an external shelter 3 having a roof 4 and an outer wall 6. The roof 4 is single pitched roof which is flat and arranged at a slant. The outer wall 6 has four rectangular walls that define a rectangular cuboid and, together with roof 4, it encloses an internal space 7. The external shelter 3 further comprises an internal support provided inside the internal space that is not shown in the figures. In the first exemplary embodiment, the internal support is a vertical bar for attaching the housing 2, but other types of support may be envisaged for other embodiments depending on the particularities of the housing 2. Fig 3 and Fig. 4 show that one the four walls of the outer wall 6, is hinged and can act as a door that is swingable between an opened position (as in the figures) and a closed position. For the sake of clarity, the hinged wall will be referred as "frontal" in this document. In this embodiment, the wall is attached by two hinges that are arranged at the bottom. Even if it is not shown in the figures, a latch is arranged at the top to act as locking means configured for locking the frontal wall when in said closed position. Other locking means may be envisaged depending for example, on the size and weight of the frontal wall.

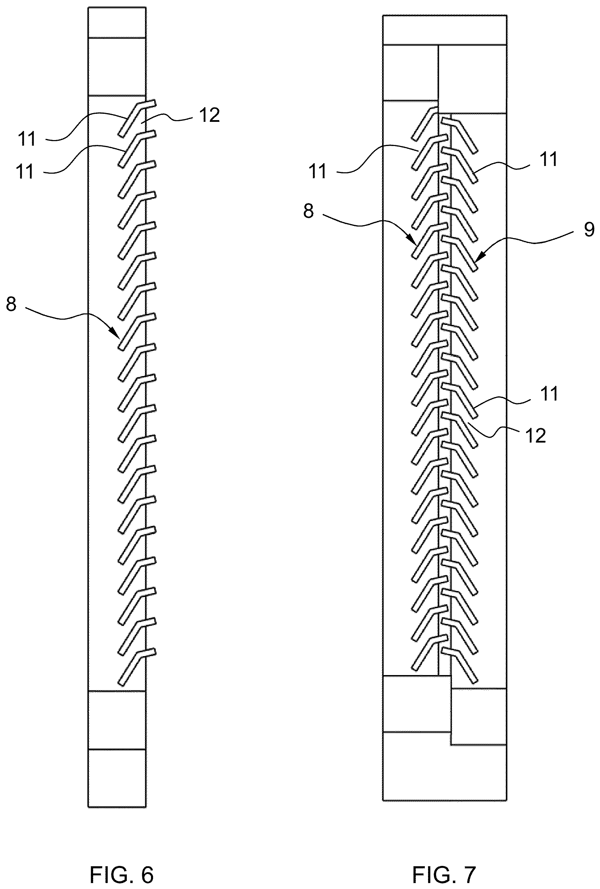

Each of the four rectangular walls has an external louver 8 which is fixed and formed by a plurality of strips 11, each strip 11 arranged horizontally and having a roll inclination, with a vertical separation 12 between each two neighbouring strips 11. The external louvers 8 are shown in Fig. 3 and Fig. 4, but the particular details of the strips 11 and their separation can be found in the section view of Fig. 6. In particular, the first exemplary embodiment of the device 1 uses a particular model of a weather instrument shelter, also called *Stevenson screen,* as the basis component for the external shelter 3, because this type of shelter is provided with the required louvers that provide the technical effects described above.

In the first embodiment, the external shelter 3, in particular, the roof 4 and outer wall 6 are made of composite plastic. However, other embodiments use different materials, for example, plastics, fiberglass, wood, plywood, oriented strand boards (OSB) o particle boards. The internal surfaces of the external shelter 3 are black and the external surfaces are white. In other embodiments, the black colour may be replaced by a dark colour, and the white colour by a clear colour. In the first exemplary embodiment, the internal space 7 has a height of 421 mm, a width of 354 mm, and a depth of 231 mm. The housing 2 has a height of 210 mm, a width of 176 mm, and a depth of 114 mm. Therefore, the internal space 7 is approximately two times the size of the housing 2 in every dimension X, Y and Z, such that the housing 2 may be adequately separated from the internal side of the outer wall 6.

Other embodiments of the device according to the invention are disclosed hereinafter. These embodiments share most of the features disclosed in the first embodiment above. Therefore, only the differentiating features will be described in detail. For the sake of brevity, common features shared with the first embodiment disclosed above will not be described again hereinbelow.

In other embodiments, the air quality related sensors may also comprise a nephelometer for obtaining measurements of PM, a photo ionization detector, PID, for obtaining measurements VOC, a solid-state gas sensor, a MEMS gas sensor, or a photoacoustic gas sensor.

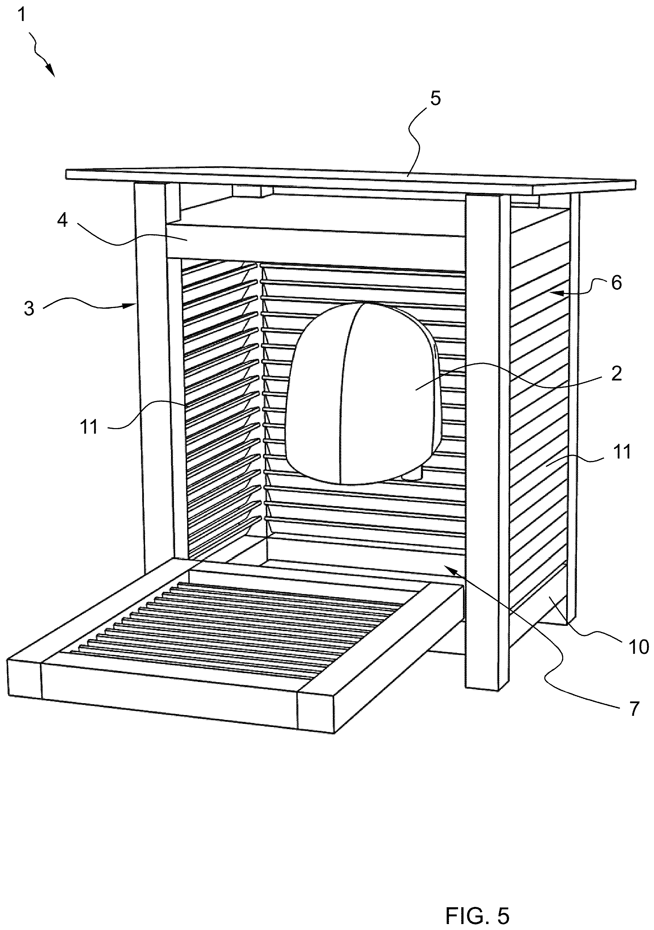

Fig. 5 is a schematic representation of a second exemplary embodiment of the device 1 wherein the external shelter 3 further comprises a base floor 10, and a further roof 5 that is provided over the roof 4. For the sake of consistency in the nomenclature, in this document, the roof 4 refers to the element enclosing the internal space 7. In the second embodiment the roof 4 is flat and horizontal, while the further roof 5 is also flat but arranged at a slant. In other embodiments, the further roof 5 has a pyramidal or conical shape.

In a third embodiment shown in the section view of Fig. 7, each of the rectangular walls of the outer wall 6 further comprises an internal louver 9. As in the case of the external louver 8, the internal louver 9 is fixed and formed by a plurality of strips 11 that are arranged horizontally and have a roll inclination, with a vertical separation 12 between two consecutive strips 11. As shown in the section view of Fig. 7, the roll inclination of the strips 11 of the internal louver 9 is contrary to the roll inclination of said strips 11 of the external louver 8.

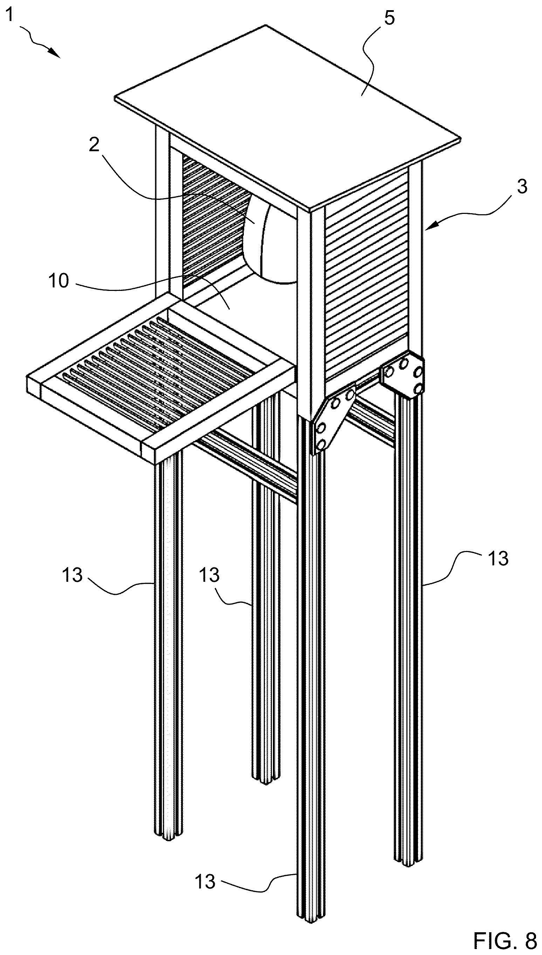

In a fourth exemplary embodiment derived from the second exemplary embodiment shown in Fig. 8, the device 1 further comprises a support for the external shelter 3. Said support is made of four legs 13, in particular, one for each corner of the external shelter 3, and each leg 13 having a length of 120 cm. The addition of legs 13 is not exclusive derivable from the second embodiment, therefore, other embodiments with at least one leg 13 may be envisaged for any of the other embodiments disclosed herein.

In a fifth exemplary embodiment derived from any of the previous embodiments and not shown in the figures, the external shelter 3 is provided with forced ventilation means, that is configured for forcing the circulation of air between the outside of the external shelter 3 and the internal space 7.

## Claims

1. Device (1) for monitoring air quality, comprising a housing (2) containing:
- at least one sensor, configured to obtain measurements of at least one component of the atmospheric composition related to air quality;
- a control means, configured to receive said measurements of said at least one sensor, and to obtain air monitoring data from said measurements; and
- a communication means, configured to send said air monitoring data to an external device;
**characterized in that** the device (1) further comprises an external shelter (3) comprising:
- a roof (4);
- an outer wall (6), comprising four rectangular walls defining a cube or cuboid, said outer wall (6) enclosing an internal space (7) covered by said roof (4); and
- an internal support, provided inside said internal space (7);
wherein each of said four rectangular walls comprises an external louver (8);
wherein said housing (2) is attached to said internal support.

2. Device (1) according to claim 1, wherein each of said rectangular walls further comprises an internal louver (9).

3. Device (1) according to any one of the claims 1 or 2, wherein said external shelter (3) comprises a further roof (5), provided over said roof (4).

4. Device (1) according to claim 3, wherein said further roof (5) is flat and arranged at a slant, has a pyramidal shape or a has conical shape.

5. Device (1) according to any one of the claims 1 to 4, further comprising a support for said external shelter (3), said support preferably comprising at least one leg (13) and more preferably four legs (13), preferably said at least one leg (13) having a length of 100 to 160 cm, in particular 120 cm.

6. Device (1) according to any one of the claims 1 to 5, wherein at least one of said four walls is hinged such that it can act as a door that is swingable between an opened position and a closed position; and locking means configured for locking said wall when in said closed position.

7. Device (1) according to any one of the claims 1 to 6, wherein said internal space (7) has a height of 350 - 730 mm, a width of 230 - 700 mm, and a depth of 200 - 630 mm.

8. Device (1) according to any one of the claims 1 to 7, wherein said housing (2) has a housing height, a housing width and a housing depth, and said internal space (7) has a space height, a space width and a space depth, wherein said space height is two times said housing height, said space width is two times said housing width, and said space depth is two times said housing depth.

9. Device (1) according to any one of the claims 1 to 8, wherein said external shelter (3) is made of at least one of wood, plywood, oriented strand board, particle board, fiberglass, and plastic.

10. Device (1) according to any one of the claims 1 to 9, wherein said external shelter (3) has an internal surface facing said internal space (7), and an external surface; and wherein said external surface is white or of a clear colour.

11. Device (1) according to any one of the claims 1 to 10, wherein said external shelter (3) has an internal surface facing said internal space (7); and wherein said internal surface is black or of a dark colour.

12. Device (1) according to any of the claims 1 to 11, wherein said external shelter (3) further comprises a base floor (10).

13. Device (1) according to any of the claims 1 to 12, wherein said external louver (8) and, where appropriate, also said internal louver (9), are each formed by a plurality of strips (11) arranged horizontally and having a roll inclination, with a vertical separation (12) between strips (11).

14. Device (1) according to claim 13, wherein said external shelter (3) comprises an external louver (8) and an internal louver (9), and wherein said horizontal strips (11) of said internal louver (9) have a roll inclination which is contrary to the roll inclination of said horizontal strips (11) of said external louver (8).

15. Device (1) according to any one of the claims 1 to 14, wherein said external shelter (3) is provided with forced ventilation means, configured for forcing the circulation of air between the outside of said external shelter (3) and said internal space (7).
